(19) Europäisches Patentamt
European Patent Office
Office européen des brevets

(11) EP 0 570 136 B1

(12) **EUROPEAN PATENT SPECIFICATION**

(45) Date of publication and mention of the grant of the patent:
**12.08.1998 Bulletin 1998/33**

(51) Int. Cl.$^6$: **B01J 29/40**, B01J 29/65, C07D 201/04

(21) Application number: **93303388.8**

(22) Date of filing: **29.04.1993**

(54) **Selective surface dealumination of zeolites**

Selektive Dealuminierung von Zeolithen

Désalumination selective de zéolites

(84) Designated Contracting States:
**BE DE DK FR GB IT NL SE**

(30) Priority: **11.05.1992 US 881282**
**12.03.1993 US 30512**

(43) Date of publication of application:
**18.11.1993 Bulletin 1993/46**

(73) Proprietor:
**MOBIL OIL CORPORATION**
**New York New York 10017 (US)**

(72) Inventors:
- **Apelian, Minas Robert**
**Vincentown, New Jersey 08088 (US)**
- **Bell, Weldon Kay**
**Pennington, New Jersey 08534 (US)**
- **Fung, Shiu Lun Anthony**
**Wilmington, Delaware 19810 (US)**
- **Haag, Werner Otto**
**Lawrenceville, New Jersey 08648 (US)**
- **Venkat, Chaya Rao**
**Princeton, New Jersey 08540 (US)**

(74) Representative: **Kador & Partner**
**Corneliusstrasse 15**
**80469 München (DE)**

(56) References cited:

| | |
|---|---|
| EP-A- 553 562 | EP-A- 0 171 981 |
| EP-A- 0 234 088 | FR-A- 2 365 520 |
| US-A- 3 691 099 | US-A- 5 200 168 |

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).



Printed by Xerox (UK) Business Services
2.16.3/3.4

## Description

This invention is directed to a process for the selective surface dealumination of zeolites

Zeolitic materials, both natural and synthetic, have been demonstrated to have catalytic properties for various types of hydrocarbon conversion and chemical processing. It is often advantageous to dealuminate these materials in order to improve their process performance. Performance measures include product selectivity, product quality and catalyst stability.

Conventional techniques for zeolite dealumination include hydrothermal treatment, mineral acid treatment with HCl, $HNO_3$, and $H_2SO_4$, and chemical treatment with $SiCl_4$ or ethylenediaminetetraacetic acid (EDTA). The treatments, however, do not exhibit selectivity to the zeolite crystal surface.

U.S. Patent 3,442,795 to Kerr et al. describes a process for preparing highly siliceous zeolite-type materials from crystalline aluminosilicates by means of a solvolysis, e.g. hydrolysis, followed by a chelation. In this process, the acid form of a zeolite is subjected to hydrolysis, to remove aluminum from the aluminosilicate. The aluminum can then be physically separated from the aluminosilicate by the use of complexing or chelating agents such as EDTA or carboxylic acid, to form aluminum complexes that are readily removable from the aluminosilicate. The examples are directed to the use of EDTA to remove alumina.

EP 0 259 526 B1 discloses the use of dealumination in producing ECR-17. The preferred dealumination method involves a combination of steam treatment and acid leaching, or chemical treatments with silicon halides. The acid used is preferably a mineral acid, such as HCl, $HNO_3$ or $H_2SO_4$, but may also be weaker acids such as formic, acetic, citric, oxalic, tartaric acids and the like.

U.S. Patent 4,388,177 discloses modifying the shape selectivity of natural ferrierite by treating with oxalic acid to impart catalytic activity.

U.S. Patent 4,088,605 discloses a crystalline aluminosilicate zeolite containing an aluminum-free outer shell prepared by initiating the crystallization in a crystallization medium and then altering the crystallization medium to eliminate the aluminum therein. This can be accomplished by a total replacement of the reaction mixture or by complexing from the original reaction mixture any remaining aluminum ion with reagents such as gluconic acid, tartaric acid, nitrilotriacetic acid or EDTA.

Non-selective reactions on the surface acid sites of the zeolite are generally undesirable. These non-selective reactions often lead to lower product yield and/or inferior product characteristics. To minimize the incidence of undesirable reactions occuring on the surface of the zeolite catalyst methods have been used to reduce or eliminate surface acidity by extraction with bulky reagents or by surface poisoning.

Zeolite modification by exchange and similar technology with large cations such as $N^+$ and $P^+$ and large branched compounds such as polyamines and the like is described in U.S. Patent No. 4,101,595. Bulky phenolic and silicating zeolite surface modifying agents are described in U.S. Patent Nos. 4,100,215 and 4,002,697, respectively. The surface acidity of the zeolite can be eliminated or reduced by treatment with bulky dialkylamine reagents as described in U.S. Patent Nos. 4,520,221 and 4,568,786.

U.S. Patent 4,716,135 discloses zeolite catalysts can be surface inactivated by cofeeding a sterically hindered base or organophosphorus compound. U.S. Patent 5,080,878 discloses modifying a crystalline aluminosilicate zeolite with a fluorosilicate salt to extract surface zeolite aluminum which is replaced by silicon. U.S. Patent 5,043,307 discloses modifying a crystalline aluminosilicate zeolite by steaming as synthesized zeolite containing organic template material and then contacting the zeolite in the ammonium, alkali metal, or hydrogen form with a dealuminizing agent which forms a water soluble complex with aluminum. These methods, however, often increase the complexity and operability of the process.

Limiting surface acidity is desirable for preventing undesired reactions on the zeolite surface which are not subject to the shape selective constraints imposed on those reactions occurring within the zeolite interior. However reducing the surface acidity will generally effect a reduction in overall activity of the zeolite. An object of the present invention is to provide a process of treating zeolites with a C.I. >1 so effect a reduction in the surface acidity of the zeolite without a significant reduction in its overall activity.

Epsilon caprolactam, usually referred to simply as "caprolactam", is a large volume commodity chemical used as a monomer in the production of the commercially important Nylon-6. Although routes to the precursor cyclohexanone oxime vary, all commercial caprolactam production makes use of a Beckmann rearrangement of the oxime. The commercial reaction is carried out in a batch operation in oleum ($H_2SO_4SO_3$) solution. The recovery step in this technology employs an ammonium hydroxide neutralization of the resulting caprolactam-oleum solution, a process generating two moles of by-product ammonium sulfate per mole of product. The sulfate has some value as a low grade fertilizer, but its recovery and/or disposal can add substantial cost to Nylon-6 production. Attempts have been made to circumvent the use of oleum and carry out the reaction in the gas phase, thereby eliminating the undesirable by-product.

A number of patents and publications have appeared which describe such heterogeneous gas phase conversions. Examples of these include U.S. Patent 3,503,958 to P.S. Landis, which describes and claims such conversion using a

zeolite such as hydrogen Y; U.S. Patent 3,016,375 which uses as catalyst polyphosphoric acid; and U.S. Patent 4,359,421 to Bell et al. which uses as catalyst a zeolite having a silica to alumina ratio of at least 12 and a Constraint Index of 1 to 12.

U.S. Patent 4,582,815 to Bowes describes and claims a method for preparing binder-free and silica-bonded extrudates of zeolites, including ZSM-5.

U.S. Patent 4,697,010 to McMahon discloses a process for the catalyzed conversion of cyclohexanone oxime to caprolactam over a catalyst composition comprising the hydrogen form of crystals having the structure of ZSM-5 made in the presence of a boron source.

U.S. Patent 4,709,024 to Sato et al. discloses a process for the production of epsilon caprolactam using a crystalline aluminosilicate catalyst having specific Si/Al atomic ratio and a specific acid amount of external surface.

U.S. Patent 4,927,924 to Bell et al. discloses synthesis of caprolactam using a catalyst subjected to steam treatment to reduce its Alpha Value.

The desired rearrangement of cyclohexanone oxime (I) to caprolactam (II) is believed to occur via a protonated intermediate (not shown), according to Equation A.

$(CH_2)_5$ C=NOH (I)

$(CH_2)_5$ N-H / C=O (II)

(Eq.A)

In addition, however, two major side reactions occur, one with the formation of 5-cyanopentene (III) and water (Equation B), and the other forming cyclohexanone oxime (IV) (Equation C).

$(CH_2)_5$ C=NOH (I)

$(CH_2)_3$-C≡N / CH=$CH_2$ (III) $+H_2O$

(Eq.B)

(Eq.C)

$(CH_2)_5$ C=NOH + $H_2O$ (I)

$(CH_2)_5$ C=O + $NH_2OH$ (IV)

In addition to (III) and (IV) other by-products of unknown structure also are formed.

Another object of the present invention is to provide a heterogenous process which is highly selective for the desired caprolactam product. It is found that the surface-dealuminated zeolites of the present invention exhibit an increased in selectivity for the conversion of cyclohexanone oxime to epsilon caprolactam. It is further found that the surface-dealuminated zeolites of the present invention exhibit improved (slower) aging when used in caprolactam synthesis.

The present invention provides a process for the selective surface dealumination of a zeolite having a Constraint Index greater than 1 comprising contacting the zeolite with a dicarboxylic acid for a sufficient time to effect at least about 40% reduction in surface acidity with less than about 50% overall dealumination.

The process of the invention is useful for the selective surface dealumination of a zeolite having a Constraint Index greater than 1.

The method by which Constraint Index is determined is described fully in U.S. Patent No. 4,016,218. Constraint Index (CI) values for some typical zeolites including some which are suitable in the process of this invention are:

| | CI (at test temperature) |
|---|---|
| ZSM-4 | 0.5 (316°C) |
| ZSM-5 | 6-8.3 (371°C-316°C) |
| ZSM-11 | 5-8.7 (371°C-316°C |
| ZSM-12 | 2.3 (316°C) |
| ZSM-20 | 0.5 (371°C) |
| ZSM-22 | 7.3 (427°C) |
| ZSM-23 | 9.1 (427°C) |
| ZSM-34 | 50 (371°C) |
| ZSM-35 | 4.5 (454°C) |
| ZSM-48 | 3.5 (538°C) |
| ZSM-50 | 2.1 (427°C) |
| MCM-22 | 1.5 (454°C) |
| TMA Offretite | 3.7 (316°C) |
| TEA Mordenite | 0.4 (316°C) |
| Clinoptilolite | 3.4 (510°C) |
| Mordenite | 0.5 (316°C) |
| REY | 0.4 (316°C) |
| Amorphous Silica-alumina | 0.6 (538°C) |
| Dealuminized Y | 0.5 (510°C) |
| Erionite | 38 (316°C) |
| Zeolite Beta | 0.6-2.0(316°C-399°C) |

Some zeolite catalysts which are useful in the process of this invention include zeolites ZSM-5, ZSM-11, ZSM-22, ZSM-23, ZSM-35, MCM-22, and MCM-49.

ZSM-5 is described in U.S. Patent No. 3,702,886; ZSM-11 is described in U.S. Patent No. 3,709,979; ZSM-22 is described in U.S. Patent 4,556,477; ZSM-23 is described in U.S. Patent No. 4,076,842; ZSM-35 is described in U.S. Patent No. 4,016,245; and MCM-22 is described in U.S. Patent No. 4,954,325. MCM-49 is described in International Publication No. WO 92/22498.

Prior to or following the selective surface dealumination process of the present invention, it may be desirable to incorporate the zeolites with another material resistant to the temperature and other conditions employed in the process. Such matrix materials include synthetic or natural substances as well as inorganic materials such as clay, silica and/or metal oxides, such as titania or zirconia. The latter may be either naturally occurring or in the form of gelatinous precipitates or gels including mixtures of silica and metal oxides. Naturally occurring clays which can be composited with the zeolite include those of the montmorillonite and kaolin families. These clays can be used in the raw state as originally mined or initially subjected to calcination, acid treatment or chemical modification. These materials, i.e. clays, oxides, etc., function, in part, as binders for the catalyst.

In addition to the foregoing materials, the zeolites may be composited with a porous matrix material, such as alumina, silica-alumina, silica-magnesia, silica-zirconia, silica-thoria, silica-beryllia, silica-titania as well as ternary compositions, such as silica-alumina-thoria, silica-alumina-zirconia, silica-alumina-magnesia, and silica-magnesia-zirconia. The matrix may be in the form of a cogel. The relative proportions of zeolite component and inorganic oxide gel matrix may vary widely with the zeolite content ranging from between 1 to 99, more usually 5 to 80, percent by weight of the composite.

Suitable dicarboxylic acids for use in the process of this invention include oxalic, malonic, succinic, glutaric, adipic, maleic, phthalic, isophthalic, terephthalic, fumaric, tartaric or mixtures thereof. Oxalic acid is preferred. The dicarboxylic acid may be used in solution, such as an aqueous dicarboxylic acid solution.

Generally, the acid solution has a concentration in the range from 0.01 to 4 M, preferably from 1 to 3 M.

The dicarboxylic acid is generally in a volume solution to volume catalyst ratio of at least 1:1, preferably at least 4:1.

Treatment time with the dicarboxylic acid solution is as long as required to provide the desired dealumination. Generally the treatment time is at least 10 minutes. Preferably, the treatment time is at least 1 hour.

The treatment temperature is generally in the range from 0°C (32°F) to about reflux. Preferably, the treatment temperature is from 15° to 93°C (60°F to 200°F), and more preferably from 49° to 82°C (120°F to 180°F).

More than one dicarboxylic acid treatment step may be employed in the process of the present invention for enhanced surface dealumination.

The dicarboxylic acid treatment of this invention may also be combined with other conventional dealumination techniques, such as steaming and chemical treatment.

The dicarboxylic acid selectively dealuminates the surface acid sites of zeolites with a C.I. >1. The presence of surface acid sites, or surface acidity, is determined by the dealkylation of tri-tertbutylbenzene (TTBB), a bulky molecule that can only react with the acid sites on the zeolite crystal surface.

Dealkylation of TTBB is a facile, reproducible method for measuring surface acidity of catalysts. External surface activity can be measured exclusive of internal activity for zeolites with pore diameters up to and including faujasite. As a test reaction dealkylation of TTBB occurs at a constant temperature in the range of from 25 to 300°C, and preferably in the range of from 200 to 260°C.

The experimental conditions for the test used herein include a temperature of 200°C and atmospheric pressure. The dealkylation of TTBB is carried out in a glass reactor (18 cm x 1 cm OD) containing an 8 g 14/30 mesh Vycor chip preheater followed by 0.1 g catalyst powder mixed with Vycor chips. The reactor is heated to 200°C in 30 ml/gm nitrogen for 30 minutes to remove impurities from the catalyst sample. Ten g/hr of TTBB disolved in toluene (7% TTBB) is injected into the reactor. The feed vaporizes as it passes through the preheator and is vapor when passing over the catalyst sample. After equilibrium is reached the nitrogen is switched to 20 ml/min hydrogen. The test is then run for 30 minutes with the reaction products collected in a cold trap.

The reaction products are analyzed by gas chromatography. The major dealkylation product is di-t-butylbenzene (DTBB). Further dealkylation to t-butylbenzene (TBB) and benzene (B) occurs but to a lesser extent.

Conversion of TTBB is calculated on a molar carbon basis. Dealkylation product weight % are each multiplied by the appropriate carbon number ratio to convert to the equivalent amount of TTBB, i.e. DTBB x 18/14, TBB x 18/10 and B x 18/6. These values are then used in the following conversion equation where asterisks indicate adjustment to the equivalence.

$$\%\ \text{Conversion} = \frac{\text{DTBB}^* + \text{TBB}^* + \text{B}^*}{\text{TTBB} + \text{DTBB}^* + \text{TBB}^* + \text{B}^*}$$

In addition, thermal background experiments using reactors filled with vycor chips only show no TTBB conversion due to Vycor chips or other reactor components.

The dicarboxylic acid treatment of this invention results in less than 50% overall dealumination, preferably less than 20% overall dealumination, and more preferably less than 10% overall dealumination with greater than 40% reduction in surface acidity, preferably greater than 50% reduction in surface acidity, and more preferably greater than 60% reduction in surface acidity.

When intended for use in the the production of caprolactam, the zeolite subjected to the surface dealumination treatment of the invention preferably has an initial alpha value of 0.1 to 50. Alpha value provides an approximate indication of the catalytic cracking activity of a catalyst compared to a standard catalyst. In particular, alpha value measures the relative rate constant (rate of normal hexane conversion per volume of catalyst per unit time) compared to that of a silica-alumina cracking catalyst taken as an Alpha of 1 (Rate Constant = 0.016 sec $^{-1}$). The Alpha Test is described in U.S. Patent 3,354,078; in the Journal of Catalysis, Vol. 4, p. 527 (1965); Vol. 6, p. 278 (1966); and Vol. 61, p. 395 (1980). The experimental conditions of the test used herein include a constant temperature of 538°C and a variable flow rate as described in detail in the Journal of Catalysis, Vol. 61, p. 395.

Conversion of cyclohexanone oxime to caprolactam using the surface-dealuminated catalyst of the present invention is desirably carried out in a conventional fixed bed reactor, although an ebullated or fluidized bed or other type of

reactor can be useful, too, with appropriate changes in the particle size and other physical attributes of the catalyst, such as attrition resistance. The reaction temperature is preferably in the range 150° to 500°C, and more preferably 200° to 400°C. Although the reaction can be carried out at atmospheric pressure, elevated pressure from 170 to 2860 kPa (10 to 400 psig), and more preferably from 450 to 2170 kPa (50 to 300 psig), is desirable.

It is contemplated that the cyclohexanone oxime feed to the process of this invention may be passed to the reactor neat, i.e., as undiluted solid, melt, or vapor, or it may be diluted with an essentially inert solvent. The use of inert solvent provides a convenient means for storing and transferring the cyclohexanone oxime to the reaction zone. The term "inert solvent" as used herein means a solvent which does not react with the oxime or its reaction product under conversion conditions, and which is not itself converted to a significant extent when contacted with the catalyst under conversion conditions. Useful solvents include the lower boiling saturated hydrocarbons such as hexane and aromatic compounds such as benzene. Regardless whether the cyclohexanone oxime feed is undiluted or dissolved in inert solvent, a carrier gas may be used with the feed to help displace reaction product and any unconverted oxime from the catalyst. Among the gases which may be used are hydrogen, nitrogen, helium, carbon monoxide, carbon dioxide, steam, and the like.

Regardless of how the cyclohexanone oxime is introduced, it is contemplated that useful conversion will be obtained at a LHSV (i.e., volumes of cyclohexanone oxime feed per volume of catalyst) within the range 0.002 to about 5.0.

The following examples illustrate the process of the present invention.

**Example 1**

Sixty-five parts by weight of ZSM-35 synthesized in accordance with U.S. Patent No. 4,016,245, and having a Constraint Index of 4.5 is mixed with 35 parts of $SiO_2$ on a dry basis. The mixture is dry mulled and formed into 1.58 mm (1/16") cylindrical extrudates. The extrudates are dried at 121°C (250°F) for 8 hours, activated and calcined in air at 538°C (1000°F) for 3 hours. The resulting catalyst referred to henceforth as Catalyst A has the following properties:

| | |
|---|---|
| Alpha Value | 102 |
| Surface Acidity | 18 |
| $Al_2O_3$, wt% | 5.7 |

**Example 2**

A sample of Catalyst A as set forth in Example 1 is treated with 2M oxalic acid at 71°C (160°F) for 1 hour. The treated sample is washed with water, dried at 121°C (250°F) for 8 hours and calcined in air at 537°C (1000°F) for 3 hours. The treatment results in 4% overall dealumination with 44% reduction in surface acidity. The oxalic acid treated catalyst has the following properties:

| | |
|---|---|
| Alpha Value | 92 |
| Surface Acidity | 10 |
| $Al_2O_3$, wt% | 5.5 |

**Example 3**

Sixty-five parts by weight of ZSM-23 synthesized in accordance with U. S. Patent No. 4,076,842 and having a Constraint Index of 9.1, is mixed with 35 parts by weight of $SiO_2$ on a dry basis. The mixture is dry mulled and formed into 1.58 mm (1/16") cylindrical extrudates. The extrudates are dried at 121°C (250°F) for 8 hours, activated and calcined in air at 537°C (1000°F) for 3 hours. The resulting catalyst henceforth referred to as Catalyst B has the following properties:

| | |
|---|---|
| Alpha Value | 25 |
| Surface Acidity | 4.6 |
| $Al_2O_3$, wt% | 1.1 |

**Example 4**

A sample of Catalyst B as set forth in Example 3 is treated with 2M oxalic acid at 71°C (160°F) for 1 hour. The treated sample is washed with water, dried at 121°C (250°F) for 8 hours and calcined in air at 537°C (1000°F) for 3 hours. The treatment results in 18% overall dealumination with 50% reduction in surface acidity. The oxalic acid treated catalyst has the following properties:

| | |
|---|---|
| Alpha Value | 26 |
| Surface Acidity | 2.1 |
| $Al_2O_3$, wt% | 0.9 |

**Example 5**

Sixty-five parts by weight ZSM-5 synthesized in accordance with U.S. Patent No. 3,702,886, and having a Constraint Index of 6.0, is mixed with 35 parts by weight of $SiO_2$ on a dry basis. The mixture is dry mulled and formed into 1.58mm (1/16") cylindrical extrudates. The extrudates are dried at 121°C (250°F) for 8 hours, activated and calcined in air at 537°C (1000°F) for 3 hours. The resulting catalyst henceforth referred to as Catalyst C has the following properties:

| | |
|---|---|
| Alpha Value | 275 |
| Surface Acidity | 18 |
| $Al_2O_3$, wt% | 1.6 |

**Example 6**

A sample of catalyst C as set forth in Example 5 is treated with 2M oxalic acid at 71°C (160°F) for 1 hour. The treated sample is washed with water, dried at 121°C (250°F) for 8 hours and calcined in air at 537°C (1000°F) for 3 hours. The treatment results in 6% overall dealumination with 94% reduction in surface acidity. The oxalic acid treated catalyst has the following properties:

| | |
|---|---|
| Alpha Value | 258 |
| Surface Acidity | <1 |
| $Al_2O_3$, wt% | 1.5 |

**Example 7**

This is a comparative example which demonstrates the dealumination of large-pore zeolites with a C.I. <1 is non-selective to the zeolite crystal surface and affects the overall activity as measured by Alpha Value.

Zeolite Beta synthesized in accordance with U.S. Patent Nos. 3,308,069 and Re 28,341 and having a Constraint Index of 0.6. Organics are removed by treating in $N_2$ at 343°C (650°F) for 3 hours followed by air calcination at 537°C (1000°F) for 6 hours. The calcined zeolite henceforth referred to as catalyst D has the following properties:

| | |
|---|---|
| Alpha Value | 400 |
| $Al_2O_3$, wt% | 4.6 |
| Surface Acidity | 59 |

**Example 8**

A sample of catalyst D as set forth in Example 7 is treated with a 2M solution of oxalic acid at a solution to catalyst ratio of 16 to 1. Treatment is conducted at 71°C (160°F) for 1 hour. The treated catalyst is dried at 121°C (250°F) overnight. The dried catalyst is calcined in air at 537°C (1000°F) for 3 hours. The treatment results in 91% overall dealumination in addition to the 92% reduction in surface acidity. The oxalic acid treated catalyst has the following properties:

| | |
|---|---|
| Alpha Value | 11 |
| $Al_2O_3$, wt% | .40 |
| Surface Acidity | 7 |

**Example 9**

Two ZSM-5 catalysts, one ZSM-5 catalyst treated with oxalic acid and one ZSM-5 catalyst untreated, were tested in a down flow tubular reactor where bed length/diameter varies from about 8 to 12. A 5 wt.% solution of hexanone oxime in benzene was charged to the reactor along with a carrier of nitrogen gas with the reaction in the middle of a three zone furnace at 300°C and the reactor exit at atmospheric pressure. Base conditions were at a cyclohexanone oxime solution feed rate of 1 LHSV and a nitrogen feed of 645 GHSV.

The ZSM-5 catalyst treated with oxalic acid gave a higher yield of caprolactam and a lower aging in the above test than the untreated ZSM-5 catalyst.

**Claims**

1. A process for the selective surface dealumination of a zeolite having a Constraint Index greater than 1 comprising contacting the zeolite with dicarboxylic acid for a sufficient time to effect at least 40% reduction in surface acidity with less than 50% overall dealumination.

2. The process of claim 1 wherein the zeolite having a Constraint Index greater than 1 is selected from ZSM-5, ZSM-11, ZSM-22, ZSM-23, ZSM-35, MCM-22, and MCM-49.

3. The process of claim 1 or claim 2 wherein the surface acidity is reduced by at least 50%; and/or the overall dealumination is less than 20%.

4. The process of any preceding claim wherein the dicarboxylic acid is in solution, preferably an aqueous dicarboxylic acid solution.

5. The process of claim 4 wherein the solution of dicarboxylic acid is at a volume ratio of solution to catalyst of at least 1:1.

6. The process of any preceding claim wherein the dicarboxylic acid is in a concentration in the range of from 0.01 to 4M.

7. The process of any preceding claim wherein the dicarboxylic acid is selected from oxalic, malonic, succinic, glutaric, adipic, maleic, phthalic, isophthalic, terephthalic, fumaric, tartaric acid and mixtures thereof.

8. The process of any preceding claim wherein the contacting is for a time of at least 10 minutes; and/or the contacting is at a temperature in the range of from 15°C to 93°C.

## Patentansprüche

1. Verfahren zur selektiven Dealuminierung der Oberfläche eines Zeoliths mit einem Zwangsindex von mehr als 1, bei dem der Zeolith ausreichend lange mit Dicarbonsäure in Kontakt gebracht wird, damit eine zumindest 40%-ige Verringerung der Oberflächenacidität bei einer Gesamtdealuminierung von weniger als 50% erfolgt.

2. Verfahren nach Anspruch 1, wobei der Zeolith mit einem Zwangsindex von mehr als 1 aus ZSM-5, ZSM-11, ZSM-22, ZSM-23, ZSM-35, MCM-22 und MCM-49 ausgewählt ist.

3. Verfahren nach Anspruch 1 oder Anspruch 2, wobei die Oberflächenacidität um mindestens 50% verringert wird und/oder die Gesamtdealuminierung weniger als 20% beträgt.

4. Verfahren nach einem der vorstehenden Ansprüche, wobei die Dicarbonsäure in Lösung, vorzugsweise als wässrige Dicarbonsäurelösung vorliegt.

5. Verfahren nach Anspruch 4, wobei die Dicarbonsäurelösung ein Volumenverhältnis von Lösung zu Katalysator von mindestens 1:1 hat.

6. Verfahren nach einem der vorstehenden Ansprüche, wobei die Dicarbonsäure in einer Konzentration im Bereich von 0,01 bis 4 m vorliegt.

7. Verfahren nach einem der vorstehenden Ansprüche, wobei die Dicarbonsäure aus Oxalsäure, Malonsäure, Succinsäure, Glutarsäure, Adipinsäure, Maleinsäure, Phthalsäure, Isophthalsäure, Terephthalsäure, Fumarsäure, Tartarsäure und Mischungen davon ausgewählt ist.

8. Verfahren nach einem der vorstehenden Ansprüche, wobei der Kontakt mindestens 10 Minuten erfolgt und/oder der Kontakt bei einer Temperatur von 15 bis 93°C vorgenommen wird.

## Revendications

1. Un procédé de désaluminisation superficielle sélective d'une zéolite ayant un indice de contrainte supérieur à 1 comprenant la mise en contact de la zéolite avec un acide dicarboxylique pendant une durée suffisante Pour obtenir une diminution d'au moins 40% de l'acidité de surface avec moins de 50% de désaluminisation en tout.

2. Le procédé selon la revendication 1, dans lequel la zéolite ayant un indice de contrainte supérieur à 1 est choisie parmi ZSM-5, ZSM-11, ZSM-22, ZSM-23, ZSM-35, MCM-22 et MCM-49.

3. Le procédé selon l'une quelconque des revendications 1 ou 2, dans lequel l'acidité de surface est abaissée d'au moins 50% et/ou la désaluminisation est inférieure à 20% en tout.

4. Le procédé selon l'une quelconque des revendications précédentes dans lequel l'acide dicarboxylique en solution, de préférence, une solution aqueuse d'acide dicarboxylique.

5. Le procédé selon la revendication 4, dans lequel la solution de l'acide dicarboxylique est dans un rapport volumique solution/catalyseur d'au moins 1:1.

6. Le procédé selon l'une quelconque des revendications précédentes, dans lequel l'acide dicarboxylique est à une concentration comprise entre 0,01 et 4M.

7. Le procédé selon l'une quelconque des revendications précédentes, dans lequel l'acide dicarboxylique est choisi parmi des acides oxalique, malonique, succinique, glutarique, adipique, maléique, phtalique, isophtalique, téréphtalique, fumarique, tartrique et leurs mélanges.

8. Le procédé selon l'une quelconque des revendications précédentes, dans lequel le temps de contact est d'au moins 10 minutes et/ou le contact est effectué à une température comprise entre 15°C et 93°C.